(19) Europäisches Patentamt / European Patent Office / Office européen des brevets

(11) **EP 3 459 439 B1**

(12) **EUROPEAN PATENT SPECIFICATION**

(45) Date of publication and mention of the grant of the patent:
**23.08.2023 Bulletin 2023/34**

(21) Application number: **17275148.9**

(22) Date of filing: **22.09.2017**

(51) International Patent Classification (IPC):
**A61B 5/00** $^{(2006.01)}$    **G06F 3/01** $^{(2006.01)}$
**H04B 7/0417** $^{(2017.01)}$    **H04L 25/02** $^{(2006.01)}$

(52) Cooperative Patent Classification (CPC):
**A61B 5/7264; G06F 3/017; H04B 7/0417;**
**H04L 25/0248;** G06F 2218/10; G16H 50/20

(54) **PRESENCE OR ACTIVITY DETECTION**

ANWESENHEITS- ODER AKTIVITÄTSDETEKTION

DÉTECTION D'ACTIVITÉ OU DE PRESENCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.03.2019 Bulletin 2019/13**

(73) Proprietor: **Nokia Technologies Oy**
**02610 Espoo (FI)**

(72) Inventors:
- **ALLOULAH, Mohammed**
  **Cambridge, CB3 0FA (GB)**
- **KAWSAR, Fahim**
  **Cambridge, CB3 0FA (GB)**
- **MIN, Chulhong**
  **Cambridge, CB3 0FA (GB)**

(74) Representative: **Whiting, Gary et al**
**Venner Shipley LLP**
**5 Stirling House**
**Stirling Road**
**The Surrey Research Park**
**Guildford GU2 7RF (GB)**

(56) References cited:
- **WANG WEI ET AL: "Device-Free Human Activity Recognition Using Commercial WiFi Devices", IEEE JOURNAL ON SELECTED AREAS IN COMMUNICATIONS, IEEE SERVICE CENTER, PISCATAWAY, US, vol. 35, no. 5, 1 May 2017 (2017-05-01), pages 1118-1131, XP011649902, ISSN: 0733-8716, DOI: 10.1109/JSAC.2017.2679658 [retrieved on 2017-05-23]**
- **FADEL ADIB ET AL: "See through walls with WiFi!", SIGCOMM, ACM, 2 PENN PLAZA, SUITE 701 NEW YORK NY 10121-0701 USA, 27 August 2013 (2013-08-27), pages 75-86, XP058030656, DOI: 10.1145/2486001.2486039 ISBN: 978-1-4503-2056-6**
- **YUNZE ZENG ET AL: "Your AP knows how you move", HOT TOPICS IN WIRELESS, ACM, 2 PENN PLAZA, SUITE 701 NEW YORK NY 10121-0701 USA, 11 September 2014 (2014-09-11), pages 49-54, XP058056120, DOI: 10.1145/2643614.2643620 ISBN: 978-1-4503-3076-3**
- **Alan V. Oppenheim ET AL: "Discrete-Time Signal Processing, Second Edition" In: "Discrete-Time Signal Processing, Second Edition", 1 January 1999 (1999-01-01), Prentice Hall International, Inc., Upper Saddle River, New Jersey 07458, XP055045538, pages 796-801, * chapter 4.6.1 ***

EP 3 459 439 B1

**Description**

**FIELD**

The present specification relates to the use of signal processing techniques to infer context information (such as activity or presence) from wireless communication signals.

**BACKGROUND**

Systems that infer additional context (e.g. user behaviour) from wireless communication signals are known. Environmental dependence issues typically result in extensive in-situ training requirements in order to provide reasonable performance from such systems.

Wang Wei et. al. "Device-Free Human Activity Recognition Using Commercial WiFi Devices" (IEEE Journal on Selected Areas in Communications, Vol. 35, No. 5, May 2017, pages 1118-1131) describes Channel State Information (CSI)-based human Activity Recognition and Monitoring system (CARM). CARM is based on two theoretical models. First, a CSI-speed model that quantifies the relation between CSI dynamics and human movement speeds. Second, a CSI-activity model that quantifies the relation between human movement speeds and human activities.

**SUMMARY**

In a first aspect this specification describes a method as set out in claim 1.

Generating the output indicative of presence or activity may include generating an indication of a number of humans within the defined space. Alternatively, or in addition, generating the output indicative of presence or activity may include generating an indication of an activity level of humans within the defined space.

The channel impulse or frequency response data may be generated from channel state information for the wireless transmission system. The said channel state information may be collected. Further, the said channel state information may be collated into the multi-dimensional array or tensor.

The output indicative of presence or activity may be generated using machine learning tools.

The first aspect may include defining said defined period.

The first aspect may include generating a machine learning model.

Extracting the receive-side, transmit-side and delay-spread eigenvectors may comprise selecting eigenvectors having the strongest eigenvalues from the eigen-decomposed correlation matrices.

The statistical channel correlation matrices may be generated by tensor unfolding.

At least some of the differential unitarity measures may be generated using receive-side, transmit-side and delay-spread eigenvectors at adjacent time points.

At least some of the differential unitarity measures may be generated using receive-side, transmit-side and delay-spread eigenvectors at non-adjacent time points.

In a second aspect, this specification describes an apparatus configured to perform any method as described with reference to the first aspect.

In a third aspect, this specification describes computer-readable instructions which, when executed by computing apparatus, cause the computing apparatus to perform any method as described with reference to the first aspect.

In a fourth aspect, this specification describes a computer-readable medium having computer-readable code stored thereon, the computer readable code, when executed by at least one processor, causes performance of: generating statistical channel correlation matrices from a multi-dimensional array or tensor describing a channel impulse or frequency response at a transmit-side, receive-side and across a delay-spread of a multiple-input, multi-output wireless transmission system for a defined period; eigen-decomposing said correlation matrices; extracting receive-side, transmit-side and delay-spread eigenvectors from the eigen-decomposed correlation matrices; generating differential unitarity measures using receive-side, transmit-side and delay-spread eigenvectors at different time points, wherein generating differential unitarity measures comprises generating an indication of changes in unitarity of respective eigenbases; and generating an output indicative of presence or activity within a defined space based on said differential unitarity measures.

In a fifth aspect, this specification describes an apparatus comprising: at least one processor; and at least one memory including computer program code which, when executed by the at least one processor, causes the apparatus to: generate statistical channel correlation matrices from a multi-dimensional array or tensor describing a channel impulse or frequency response at a transmit-side, receive-side and across a delay-spread of a multiple-input, multi-output wireless transmission system for a defined period; eigen-decompose said correlation matrices; extract receive-side, transmit-side and delay-spread eigenvectors from the eigen-decomposed correlation matrices; generate differential unitarity measures using receive-side, transmit-side and delay-spread eigenvectors at different time points, wherein generating differential unitarity measures comprises generating an indication of changes in unitary of respective eigenbases; and generate an output indicative of presence or activity within a defined space based on said differential unitarity measures.

In a sixth aspect, the specification describes an apparatus comprising: means for generating statistical channel correlation matrices from a multi-dimensional array or tensor describing a channel impulse or frequency response at a transmit-side, receive-side and across a delay-spread of a multiple-input, multi-output wireless transmission system for a defined period; means for eigen-decomposing said correlation matrices; means for extracting receive-side, transmit-side and delay-spread eigenvectors from the eigen-decomposed correlation matrices; means for generating differential unitarity measures using receive-side, transmit-side and delay-spread eigenvectors at different time points, wherein generating differential unitarity measures comprises generating an indication of changes in unitarity of respective eigenbases; and means for generating an output indicative of presence or activity within a defined space based on said differential unitarity measures.

## BRIEF DESCRIPTION OF THE DRAWINGS

Example embodiments will now be described, by way of non-limiting example, with reference to the following schematic drawings, in which:

Figure 1 is a block diagram of an exemplary wireless communication system;
Figure 2 is a flow chart showing an algorithm in accordance with an exemplary embodiment;
Figure 3 is a plan view of an exemplary implementation of an embodiment;
Figures 4 and 5 show first (receive-side) cumulative distribution function results for the system shown in Figure 3;
Figures 6 and 7 show second (delayline) cumulative distribution function results for the system shown in Figure 3;
Figure 8 shows phase scatter results for the system of Figure 3;
Figures 9 and 10 show timelines used to explain features of exemplary embodiments; and
Figure 11 is a block diagram of a system in accordance with an exemplary embodiment.

## DETAILED DESCRIPTION

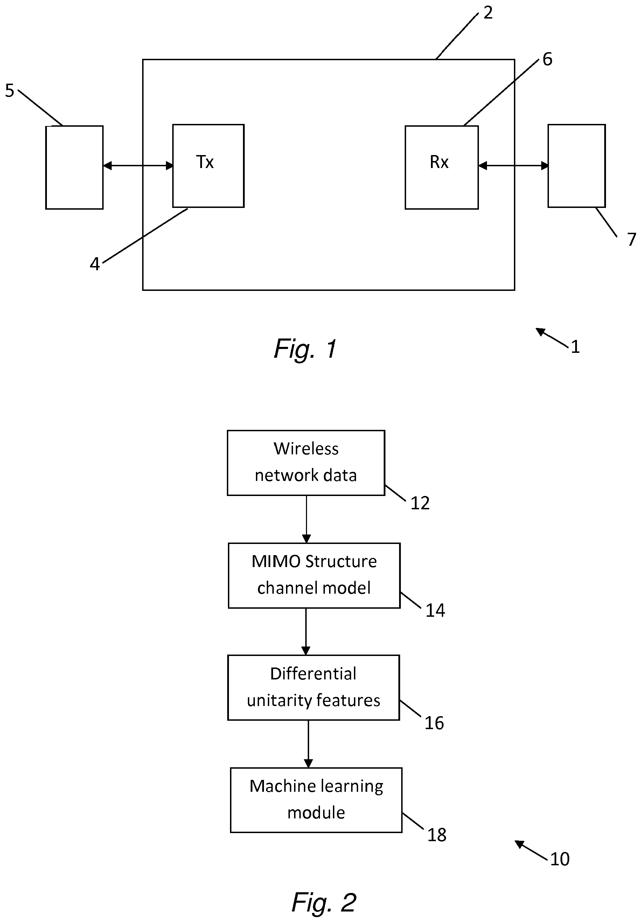

Figure 1 is a highly schematic block diagram of an exemplary wireless communication system, indicated generally by the reference numeral 1, in which exemplary embodiments described below may be implemented. The system 1 includes a space, indicated generally by the reference numeral 2, within which one or more human occupants may be present. The space 2 may, for example, be a room within a building.

The system 1 includes a multiple-input multiple-output (MIMO) transmitter 4 and a MIMO receiver 6 within the space 2. The MIMO transmitter 4 is connected to a MIMO transmitter controller 5; similarly, the MIMO receiver 6 is connected to a MIMO receiver controller 7.

The exemplary embodiments herein seek to make use of the structured MIMO channel model in order to extract metrics, such as metrics indicative of human presence or activity (for example, the presence of one or more humans within the space 2 of the system 1 described above).

The propagation of radio waves in a physical environment is governed by four phenomena: free-space pathloss, reflection, diffraction, and scattering. Sacrificing preciseness in favour of brevity, it is customary in the literature to lump as 'scatterers' any object that either reflects, diffracts, and/or scatters radio energy.

In an environment, the exact number of scatterers, their position, and electromagnetic properties at a given frequency band determine fully the conditions of wireless propagation. Given infinite computational power, electromagnetic-environmental properties can be captured in a verbose model in order to solve Maxwell equations at each receiver antenna. Such physical medium description is one extreme way to capture the nuances of the physicality of the environment.

The aim of the structured MIMO model described herein is to capture the correlation in space, time, and delay-spread (i.e. frequency-selectivity) within a MIMO channel snapshot (i.e. tensor). The second-order statistics of MIMO channels (i.e. multidimensional covariances) are described as higher-order tensors. The eigendecomposition of these MIMO tensors distils the multidimensional channel correlations into vector components. These components can be thought of as link-end excitations of the MIMO channel, and as such wholly encapsulate wireless operational conditions.

By considering MIMO channel second-order statistics (i.e. joint correlations rather than absolute-sense SISO channel dynamics), a differential view of the channel ensemble can be obtained which lends itself to a channel tracking formulation that is minimally dependent on the environment-specific radio properties. Such formulation allows for tracking of the channel dynamics arising from changes in the generative subspace (i.e. eigenmodes) while de-emphasising those shaped by the environment. Mathematical modelling inaccuracies aside, subspace tracking can therefore be viewed as an environment-invariant method for inferring human-induced channel variabilities. In this way, human presence or activity can be inferred.

It should also be noted that the use of existing wireless communications signals makes the concepts described herein backward compatible in many circumstances.

Figure 2 is a flow chart showing an algorithm, indicated generally by the reference numeral 10, in accordance with an exemplary embodiment.

The algorithm 10 starts at operation 12 where data related to a wireless network are obtained. As described in detail below, the wireless data are the relevant channel state information (CSI) data.

Next, at operation 14, the wireless data are matched with the relevant MIMO structured channel model. As described in detail below, this operation is used to compute the eigenbases for the channel based on the received data.

The algorithm then moves to operation 16, where so-called differential unitarity features are determined. As described further below, this operation determines the extent to which the differential unitarity measurement changes with time. If so, this may be indicative of the presence or activity (e.g. human presence or activity) within the relevant space.

Finally, at operation 18, a machine learning module is used to make sense of the data output by operation 16. Note that the machine learning module could take many different forms. The machine learning module could be a long short-term memory (LSTM) recurrent neural network capable of extracting patterns embedded in the history of the differential changes of the eigenbases. Further, it would be possible to implement the methods and systems described herein without using a machine learning module using a fully-modelled approach subject to the required performance-complexity-resource mode of operation.

Thus, the algorithm 10 uses the channel model of the wireless channel (determined at operation 14), which has the property of allowing the factoring out of the environmental coupling coefficients from the signal subspace on which human-induced signal fading acts. This allows the determination of robust features (i.e. differential unitarity features - operation 16) indicative of human (or other) presence or activity, which are used as input to a machine-learning model for activity recognition/presence detection. Accordingly, in at least some embodiments, the need for in situ training of the machine learning model is relatively limited.

The algorithm 10 might be used to detect human presence or human activity. Activity detection might, for example, track the profile of differential unitary within a designated window of observation which could involve both magnitude and phase signal behaviour. Further, the algorithm 10 could be used to detect other presence or activity. For example, the presence of animals (such as farm animals) could be detected.

Environmental factors that might affect the wireless channel might include the presence of a tree inside the room 2 in the system 1 described above, or the presence of a metal filing cabinet within the space 2. By making use of eigenbases and differential unitarity features (as described further below), the algorithm 10 seeks to identify data that are less prone to such environmental factors.

The structured MIMO channel model referred to in operation 14 generates three orthonormal bases for the channel tensor: receiver-side, transmitter-side, and across the delayspread.

To this end, let $H_{(n)}$ be the nth matrix unfolding of tensor H. The three tensor unfoldings $H_{(1)}$, $H_{(2)}$ and $H_{(3)}$ describe the channel behaviour in SIMO (single-input, multiple output), MISO (multiple-input, single output), and delay-spread, respectively.

$$\mathbf{H}_{(1)} = \begin{bmatrix} h_{111} & \cdots & h_{11D} & h_{121} & \cdots & h_{12D} & \cdots & h_{1N_{Tx}1} & \cdots & h_{1N_{Tx}D} \\ \vdots & \ddots & \vdots & \vdots & \ddots & \vdots & & \vdots & \ddots & \vdots \\ h_{N_{Rx}11} & \cdots & h_{N_{Rx}1D} & h_{N_{Rx}2D} & \cdots & h_{N_{Rx}2D} & \cdots & h_{N_{Rx}N_{Tx}1} & \cdots & h_{N_{Rx}N_{Tx}D} \end{bmatrix}$$

$$\mathbf{H}_{(2)} = \begin{bmatrix} h_{111} & \cdots & h_{N_{Rx}11} & h_{112} & \cdots & h_{N_{Rx}12} & \cdots & h_{11D} & \cdots & h_{N_{Rx}1D} \\ \vdots & \ddots & \vdots & \vdots & \ddots & \vdots & & \vdots & \ddots & \vdots \\ h_{1N_{Tx}1} & \cdots & h_{N_{Rx}N_{Tx}1} & h_{1N_{Tx}2} & \cdots & h_{N_{Rx}N_{Tx}2} & \cdots & h_{1N_{Tx}D} & \cdots & h_{N_{Rx}N_{Tx}D} \end{bmatrix}$$

$$\mathbf{H}_{(3)} = \begin{bmatrix} h_{111} & \cdots & h_{1N_{Tx}1} & h_{211} & \cdots & h_{2N_{Tx}1} & \cdots & h_{N_{Rx}11} & \cdots & h_{N_{Rx}N_{Tx}1} \\ \vdots & \ddots & \vdots & \vdots & \ddots & \vdots & & \vdots & \ddots & \vdots \\ h_{11D} & \cdots & h_{1N_{Tx}D} & h_{21D} & \cdots & h_{2N_{Tx}D} & \cdots & h_{N_{Rx}1D} & \cdots & h_{N_{Rx}N_{Tx}D} \end{bmatrix}$$

The receive-side correlation matrix $\mathbf{R}_{\mathrm{Rx}} \in \mathbb{C}^{N_{\mathrm{Rx}} \times N_{\mathrm{Rx}}}$ is then computed as

$$\mathbf{R}_{\mathrm{Rx}} = \mathbf{E}\left\{\mathbf{H}_{(1)}\mathbf{H}_{(1)}^{H}\right\}$$

Similarly, the transmit-side correlation matrix $\mathbf{R}_{\mathrm{Tx}} \in \mathbb{C}^{N_{\mathrm{Tx}} \times N_{\mathrm{Tx}}}$ is

$$\mathbf{R}_{\mathrm{Tx}} = \mathbf{E}\left\{\mathbf{H}_{(2)}\mathbf{H}_{(2)}^{H}\right\}$$

And the delayspread correlation matrix $\mathbf{R}_{\mathrm{Del}} \in \mathbb{C}^{D \times D}$ is

$$\mathbf{R}_{\mathrm{Del}} = \mathbf{E}\left\{\mathbf{H}_{(3)}\mathbf{H}_{(3)}^{H}\right\}$$

Eigendecomposition is then applied to the three one-sided correlation matrices in order to extract the channel eigenbases in space (receive and transmit dimensions) and in delayspread according to:

$$\mathbf{R}_{\mathrm{Rx}} = \sum_{i=1}^{N_{\mathrm{Rx}}} \lambda_{\mathrm{Rx},i}\mathbf{u}_{\mathrm{Rx},i}\mathbf{u}_{\mathrm{Rx},i}^{H} = \mathbf{U}_{\mathrm{Rx}}\Lambda_{\mathrm{Rx}}\mathbf{U}_{\mathrm{Rx}}^{H}$$

$$\mathbf{R}_{\mathrm{Tx}} = \sum_{j=1}^{N_{\mathrm{Tx}}} \lambda_{\mathrm{Tx},j}\mathbf{u}_{\mathrm{Tx},j}\mathbf{u}_{\mathrm{Tx},j}^{H} = \mathbf{U}_{\mathrm{Tx}}\Lambda_{\mathrm{Tx}}\mathbf{U}_{\mathrm{Tx}}^{H}$$

$$\mathbf{R}_{\mathrm{Del}} = \sum_{k=1}^{D} \lambda_{\mathrm{Del},k}\mathbf{u}_{\mathrm{Del},k}\mathbf{u}_{\mathrm{Del},k}^{H} = \mathbf{U}_{\mathrm{Del}}\Lambda_{\mathrm{Del}}\mathbf{U}_{\mathrm{Del}}^{H}$$

It should be noted that the terms "delayspread" and "delayline" can both be used to describe the relevant eigendecomposition dimension. These terms are used interchangeably in this document. It should also be noted that in embodiments operating in the frequency domain (rather than the time domain generally described in the exemplary embodiments), the delayspread would be referred to as frequency selectivity, by virtue of duality.

As described in detail below, we track the three eigenbases $\mathbf{U}_{\mathrm{Rx}}$, $\mathbf{U}_{\mathrm{Tx}}$ and $\mathbf{U}_{\mathrm{Del}}$. More specifically, we track changes in the eigenbases.

The inventors have observed that the eigendecomposition of the MIMO channel eigenmodes will produce eigenvectors that are by construction unitary, i.e.:

$$\mathbf{u}^{H}_{\mathrm{x},i}\mathbf{u}_{\mathrm{x},i} = 1$$

Therefore, in order to track the changes exerted on the channel as a result of human (or other) presence or activity, we monitor the successive changes in the unitarity of the eigenbases (referred to herein as 'differential unitarity' - see operation 16 of the algorithm 10).

To this end, we first define a stationary period in which the channel statistics are assumed to be stationary (e.g. 50ms). Channel decomposition is performed as per the formal MIMO Structured model discussed above. We then concentrate on the eigenvectors corresponding to the largest (i.e. strongest) eigenvalues owing to their superior signal-to-noise ratio (SNR). In a static environment, we expect the differential unitarity to be near perfect (i.e. very close to 1) owing to the unchanging statistics of the MIMO channel. Depending on the severity of statistical channel changes across two successive stationary periods, a mismatch in the decomposed bases will translate into a deviation in how unitary their eigenvectors will remain differentially from one period to the next. If we denote by $\mathbf{u}_{\mathrm{Rx,i}}[t]$ the ith receive eigenvector at time t, then the differential unitarity $\hat{\mathbf{u}}_{\mathrm{Rx,i}}[t]$ between time t and t-1 is formulated by:

$$\hat{u}_{\mathrm{Rx},i}[t] = \mathbf{u}_{\mathrm{Rx},i}^{H}[t]\, \mathbf{u}_{\mathrm{Rx},i}[t-1]$$

Similar formulation is extended to delayspread and transmit eigenbases:

$$\hat{u}_{\mathrm{Del},i}[t] = \mathbf{u}_{\mathrm{Del},i}^{H}[t]\, \mathbf{u}_{\mathrm{Del},i}[t-1]$$

$$\hat{u}_{\mathrm{Tx},i}[t] = \mathbf{u}_{\mathrm{Tx},i}^{H}[t]\, \mathbf{u}_{\mathrm{Tx},i}[t-1]$$

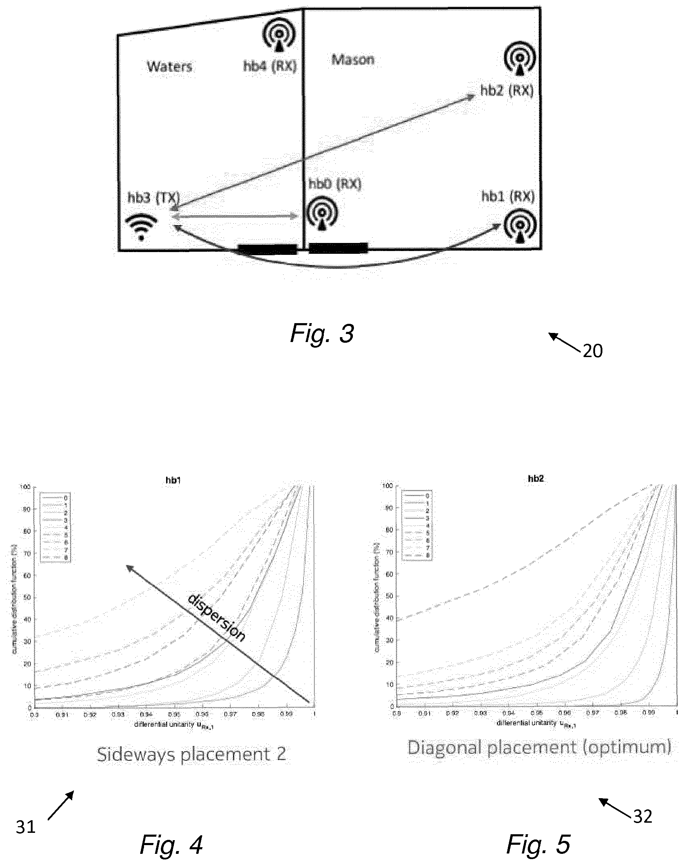

It has been found that human presence introduces dynamic channel changes that manifest themselves in significant dispersion in the differential unitarity measures, both in magnitude and phase. This is discussed further below with reference to Figures 3 to 8.

It can be shown that the synthesis equation which allows for the generation of channel tensors conforming to the measured spatio-temporal statistics is given by

$$\mathcal{H} = \tilde{\mathcal{W}} \times_1 \mathbf{U}_{\mathrm{Rx}} \times_2 \mathbf{U}_{\mathrm{Tx}} \times_3 \mathbf{U}_{\mathrm{Del}}$$

where $\tilde{\mathcal{W}}$ is the coupling coefficient tensor, and $U_{\mathrm{Rx}}$, $U_{\mathrm{Tx}}$ and $U_{\mathrm{Del}}$ are the decomposed eigenbases.

Noting the structure of the channel synthesis equation above, the coupling coefficients will 'combine' the decomposed eigenmodes as to realise a MIMO channel tensor instance. In spite of the coupling coefficients being indicative of channel fading and thus by extension being potentially also indicative of human-induced channel variations, the coupling coefficient is heavily 'statistically coloured' by the specific physicality of the environment (such as the tree and the metal filing cabinet referred to above). As such, in many embodiments, we choose to exclude the coupling coefficients of the MIMO Structured model from inference altogether, and hence our proposed environment-invariant differential unitarity tracking.

Figure 3 is a plan view, indicated generally by the reference numeral 20, of an exemplary implementation of an embodiment. The implementation 20 includes two meeting rooms (labelled Waters and Mason in Figure 3). A first transmitter hb3 is provided, together with three receivers (labelled hb0, hb1, hb2 and hb4). As shown in Figure 3, the receivers hbo, hb1 and hb2 are all within the room Mason, whilst the transmitter hb3 and the receiver hb4 are within an adjacent room Waters. For the purposes of the explanation below, the receivers hb1 and hb2 are referred to as the first and second receivers.

In testing of the system 20, a wireless communication network was set up using the transmitter hb3 and performance measurements were taken at the first receiver hb1 and the second receiver hb2. Measurements were taken with different numbers of people being present within the room Mason. Separate measurements were taken with 0, 1, 2, 3, 4, 5, 6, 7 and 8 people being present within the room Mason.

Figures 4 and 5 show first cumulative distribution function results of the receive-side differential unitary for the system shown in Figure 3. Figure 4 shows a first plot 31 showing results for the first receiver hb1 and Figure 5 shows a second plot 32 showing results for the second receiver hb2. The first plot 31 is described as a "sideways placement" plot since the signal path from transmitter hb3 to receiver hb1 is sideways with respect to the rooms shown in Figure 3. Similarly, the second plot 32 is described as a "diagonal placement" plot.

The plots 31 and 32 show the magnitude dispersion in the differential unitarity of the receive-side eigenbases for the respective receivers, with separate measurements made for different numbers of people within the room. It is clear from the plots 31 and 32 that the statistics of the magnitude of differential unitarity (i.e. the cumulative distribution function across the dataset) at the receiver eigenbasis corresponding to the strongest eigenvalue is generally monotonically increasing with increasing human presence across two different device placements within the meeting room Mason. This pattern of increasing differential unitarity can be readily identified by the machine learning module used in operation 18 with a relatively limited training phase.

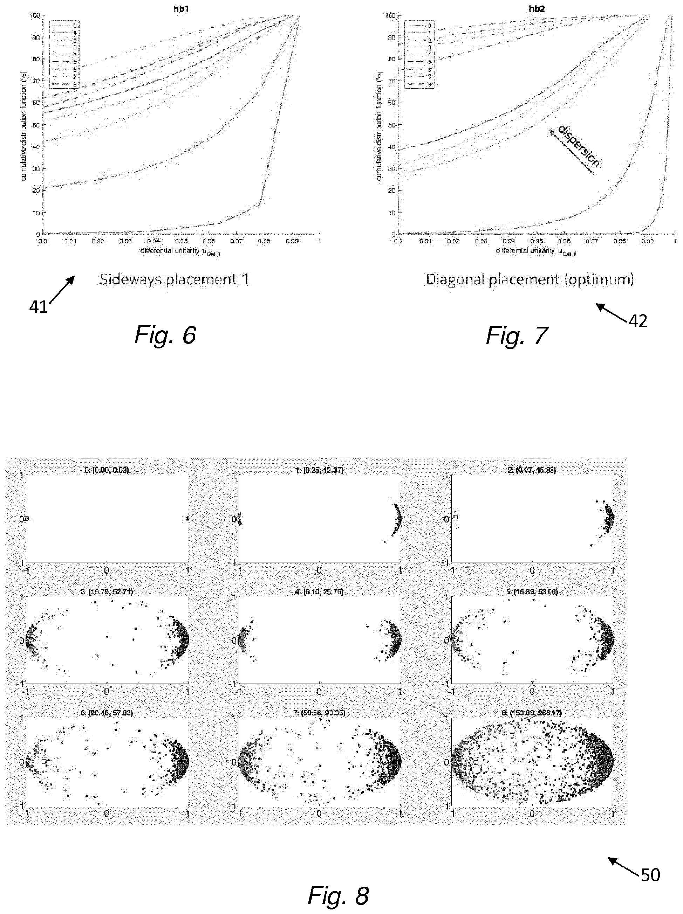

Figure 6 and 7 show second cumulative distribution function of the delayline (or delayspread) differential unitarity results for the system shown in Figure 3. Figure 6 shows a first plot 41 showing results for the receiver hb1 and Figure 7 shows a second plot 42 showing results for the second receiver hb2. The plots show the magnitude dispersion in the differential unitarity of the delayline (or delayspread) eigenbases for the respective receivers, with a separate measurement made for different numbers of people within the room. As with the plots 31 and 32 described with reference to Figures 4 and 5, it is clear from the plots 41 and 42 that the number of people has a significant impact on the delayspread differential unitarity. Again, this can be readily identified by the machine learning module used in operation 18 with a relatively limited training phase.

Figure 8 shows phase plots 50 of the receive-side (complex) differential unitary for the system of Figure 3, with the x-axes plotting the real values and the y-axes plotting the imaginary value of the complex differential unitarity measures. Each of the nine plots shows phase results with a different number of people being present within the room Mason (ranging from o people to 8 people). It is clear from the scatter plots that the number of people has a significant impact on the spread of this phase metric. This can be readily identified by the machine learning module used in operation 18 with a relatively limited training phase.

Figures 4 to 8 show three different metrics indicative of human presence that can be used by the machine learning module in the operation 18 of the algorithm 10 to predict the number of people within the room Mason. Depending on the requisite complexity-performance-resource mode of operation, the system can utilise one, two or all three metrics, with an increase in the number of metrics used generally leading to increased performance (e.g. increased reliability and sensitivity).

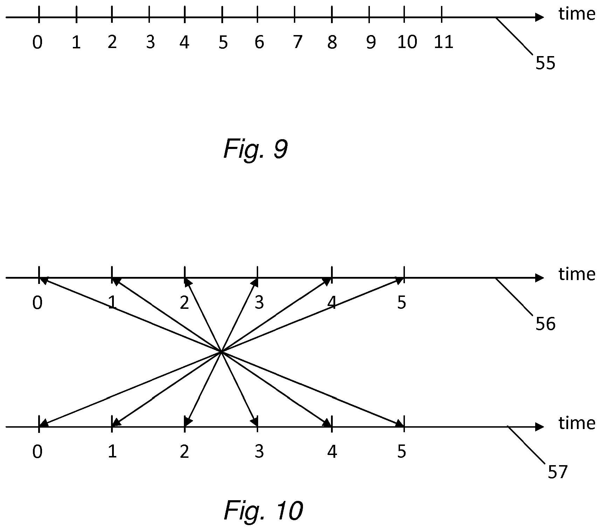

As described above, in one embodiment, a stationary period within which the channel statistics are assumed to be stationary is defined. A suggestion of 50ms was made above (although different periods could be used). Generally, the stationary period selection guideline is to choose a time interval comparable to the coherence time and coherence bandwidth of the wireless channel in line with the expected Doppler spread. Figure 9 shows a timeline 55, including a number of time points (labelled 0 to 11). The time points 0 to 11 are separated by the stationary time period (e.g. 50ms) so that the duration from t=0 to t=11 may be 550ms.

In the exemplary embodiments described above, eigendecomposition of the channel statistics is conducted at each of the time points shown in Figure 9. For one eigenbasis (e.g. receiver-side or delayspread), the strongest eigenvector is taken at each time point and compared with the strongest eigenvector of an adjacent timepoint in order to provide a measure of differential unitarity.

The differential unitarity in the embodiments described above is dependent on the values of successive eigenbases. However, in some examples, this may be omitted. For example, the different unitarity could be based on more distant eigenbases.

Figure 10 shows a first timeline 56 and a second timeline 57 similar to the timeline 55 described above. The timelines 56 and 57 are identical and show time periods t=0 to t=5.

In the arrangement shown in Figure 10, the data relating to the six time periods is buffered so that comparison can be made across a wider time window. Thus, for example, the eigebases at t=0 can be compared with those at t=5, the eigenbases at t=1 can be compared with the eigenbases at t=4 and the eigenbases at t=2 can be compared with the eigenbases at t=3. Such an arrangement may enable the changes in channel statistics to be viewed across a wider time period and may enable the rate of change of eigenvector unitarity to be determined.

The concept of differential unitarity described herein is a measure of how similar two eigenvectors, derived from two different snapshots of the channel state information, are. As described above, the eigenvectors compared to derive the differential unitarity indication do not need to be adjacent.

The timelines of described above with reference to Figures 9 and 10 are provided by way of example only. It will be readily apparent to the skilled person that alternative combinations may be provided in order for different data points to be compared.

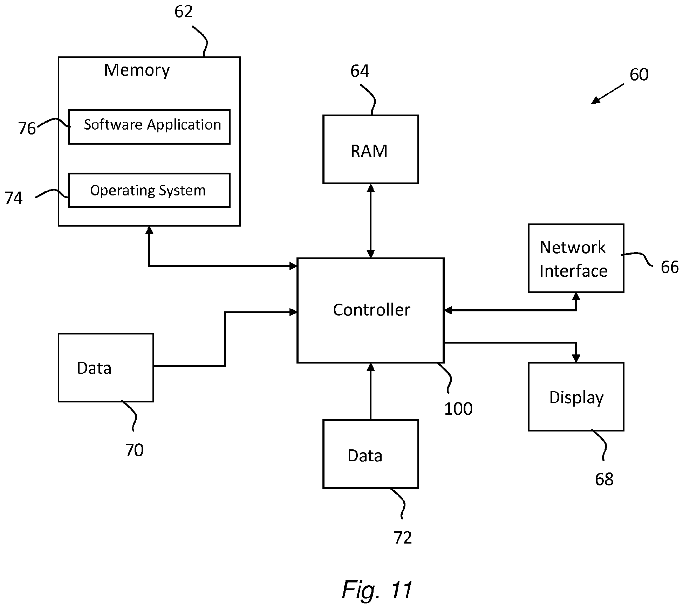

Figure 11 is a block diagram of a system, indicated generally by the reference numeral 60, that may be used in exemplary implementations of some of the embodiments described herein.

The system 60 may have a controller 100, memory 62 and RAM 64. The system 60 may comprise a network interface 66 for connection to a communications network, e.g. a modem which may be wired or wireless. The system 60 may receive data from one or more inputs (such as first data input 70 and second data input 72 shown in Figure 11). The data may, for example, relate to the channel system information (CSI) described above. The controller 100 is connected to each of the other components in order to control operation thereof.

The memory 62 may be a non-volatile memory such as read only memory (ROM), a hard disk drive (HDD) or a solid state drive (SSD). The memory 62 may store, amongst other things, an operating system 74 and one or more software applications 76 (such as an application implementing the algorithm 10 described above). The RAM 64 is used by the controller 100 for the temporary storage of data. The operating system 74 may contain code which, when executed by the controller 100 in conjunction with the RAM 64, controls operation of each of the hardware components.

The controller 100 may take any suitable form. For instance, it may be a microcontroller, plural microcontrollers, a processor, or plural processors.

In some example embodiments, the system 60 may also be associated with external software applications not stored on the memory 62. These may be applications or content data stored on a remote server device and may run partly or exclusively on the remote server device. These applications or content data may be termed cloud-hosted applications or data. The system 60 may be in communication with the remote server device in order to utilize the software application or data stored there.

Embodiments of the present invention may be implemented in software, hardware, application logic or a combination of software, hardware and application logic. The software, application logic and/or hardware may reside on memory, or any computer media. In an example embodiment, the application logic, software or an instruction set is maintained on any one of various conventional computer-readable media. In the context of this document, a "memory" or "computer-readable medium" may be any non-transitory media or means that can contain, store, communicate, propagate or transport the instructions for use by or in connection with an instruction execution system, apparatus, or device, such as a computer.

Reference to, where relevant, "computer-readable storage medium", "computer program product", "tangibly embodied computer program" etc., or a "processor" or "processing circuitry" etc. should be understood to encompass not only computers having differing architectures such as single/multi-processor architectures and sequencers/parallel architectures, but also specialised circuits such as field programmable gate arrays FPGA, application specify circuits ASIC, signal processing devices and other devices. References to computer program, instructions, code etc. should be understood to express software for a programmable processor firmware such as the programmable content of a hardware device as instructions for a processor or configured or configuration settings for a fixed function device, gate array, programmable logic device, etc.

As used in this application, the term "circuitry" refers to all of the following: (a) hardware-only circuit implementations (such as implementations in only analogue and/or digital circuitry) and (b) to combinations of circuits and software (and/or firmware), such as (as applicable): (i) to a combination of processor(s) or (ii) to portions of processor(s)/software (including digital signal processor(s)), software, and memory(ies) that work together to cause an apparatus, such as a server, to perform various functions) and (c) to circuits, such as a microprocessor(s) or a portion of a microprocessor(s), that require software or firmware for operation, even if the software or firmware is not physically present.

If desired, the different functions discussed herein may be performed in a different order and/or concurrently with each other. Furthermore, if desired, one or more of the above-described functions may be optional or may be combined. Similarly, it will also be appreciated that the flow diagram of Figure 2 is an example only and that various operations depicted therein may be omitted, reordered and/or combined.

## Claims

1. A method comprising:

generating statistical channel correlation matrices $\mathbf{R}_{TX}$, $\mathbf{R}_{RX}$, $\mathbf{R}_{Del}$, from tensor unfoldings $\mathbf{H}_{(1)}$, $\mathbf{H}_{(2)}$, $\mathbf{H}_{(3)}$ of a multi-dimensional array or tensor describing a channel impulse or frequency response at a transmit-side, receive-side and across a delay-spread of a multiple-input, multi-output wireless transmission system (1, 20) having a plurality of $N_{TX}$ transmit antennas and a plurality of $N_{RX}$ receive antennas for a defined period, wherein the channel correlation matrices comprises a transmit-side matrix, a receive-side matrix and a delay-spread matrix, wherein the tensor unfoldings describe channel behaviour in single-input multiple output, multiple-input single-output, and delay-spread respectively, as follows:

$$\mathbf{H}_{(1)} = \begin{bmatrix} h_{111} & \cdots & h_{11D} & h_{121} & \cdots & h_{12D} & \cdots & h_{1N_{Tx}1} & \cdots & h_{1N_{Tx}D} \\ \vdots & \ddots & \vdots & \vdots & \ddots & \vdots & & \vdots & \ddots & \vdots \\ h_{N_{Rx}11} & \cdots & h_{N_{Rx}1D} & h_{N_{Rx}2D} & \cdots & h_{N_{Rx}2D} & \cdots & h_{N_{Rx}N_{Tx}1} & \cdots & h_{N_{Rx}N_{Tx}D} \end{bmatrix}$$

$$\mathbf{H}_{(2)} = \begin{bmatrix} h_{111} & \cdots & h_{N_{Rx}11} & h_{112} & \cdots & h_{N_{Rx}12} & \cdots & h_{11D} & \cdots & h_{N_{Rx}1D} \\ \vdots & \ddots & \vdots & \vdots & \ddots & \vdots & & \vdots & \ddots & \vdots \\ h_{1N_{Tx}1} & \cdots & h_{N_{Rx}N_{Tx}1} & h_{1N_{Tx}2} & \cdots & h_{N_{Rx}N_{Tx}2} & \cdots & h_{1N_{Tx}D} & \cdots & h_{N_{Rx}N_{Tx}D} \end{bmatrix}$$

$$H_{(3)} = \begin{bmatrix} h_{111} & \cdots & h_{1N_{Tx}1} & h_{211} & \cdots & h_{2N_{Tx}1} & \cdots & h_{N_{Rx}11} & \cdots & h_{N_{Rx}N_{Tx}1} \\ \vdots & \ddots & \vdots & \vdots & \ddots & \vdots & & \vdots & \ddots & \vdots \\ h_{11D} & \cdots & h_{1N_{Tx}D} & h_{21D} & \cdots & h_{2N_{Tx}D} & \cdots & h_{N_{Rx}1D} & \cdots & h_{N_{Rx}N_{Tx}D} \end{bmatrix}$$

eigen-decomposing each of said correlation matrices to generate an eigen-decomposed transmit-side matrix, an eigen-decomposed receive-side matrix and an eigen-decomposed delay-spread matrix;
extracting transmit-side, receive-side and delay-spread eigenvectors from the eigen-decomposed transmit-side, receive-side and delay-spread correlation matrices;
generating (16) differential unitarity measures using transmit-side, receive-side and delay-spread eigenvectors extracted at different time points, wherein generating differential unitarity measures comprises generating an indication of changes in unitary of respective eigenbases as determined from the generated differential unitarity measures; and
generating an output indicative of presence or activity within a defined space based on said differential unitarity measures.

2. A method as claimed in claim 1, wherein generating the output indicative of presence or activity includes generating an indication of a number of humans within the defined space (2).

3. A method as claimed in claim 1 or claim 2, wherein generating the output indicative of presence or activity includes generating an indication of an activity level of humans within the defined space (2).

4. A method as claimed in any one of the preceding claims, further comprising generating channel impulse or frequency response data from channel state information for the wireless transmission system (1, 20).

5. A method as claimed in claim 4, further comprising collecting said channel state information.

6. A method according to claim 4 or claim 5, further comprising collating the channel state information into the multi-dimensional array or tensor.

7. A method as claimed in any one of the preceding claims, wherein the output indicative of presence or activity is generated using machine learning tools.

8. A method as claimed in any one of the preceding claims, further comprising defining said defined period.

9. A method as claimed in any one of the preceding claims, further comprising generating a machine learning model.

10. A method as claimed in any one of the preceding claims, wherein extracting the receive-side, transmit-side and delay-spread eigenvectors comprises selecting eigenvectors having the strongest eigenvalues from the eigen-decomposed correlation matrices.

11. A method as claimed in any one of the preceding claims, wherein the statistical channel correlation matrices are generated by tensor unfolding.

12. A method as claimed in any one of the preceding claims, wherein generating differential unitarity measures uses receive-side, transmit-side and delay-spread eigenvectors at adjacent time points.

13. A method as claimed in any one of claims 1 to 11, wherein generating differential unitarity measures comprises generating at least some differential unitarity measures using receive-side, transmit-side and delay-spread eigenvectors at non-adjacent time points.

14. A computer program comprising instructions that when executed by a computer control it to perform the method of any one of the preceding claims.

15. An apparatus configured to perform the method of any one of claims 1 to 13.

**Patentansprüche**

1. Verfahren umfassend:

   Generieren statistischer Kanalkorrelationsmatrizen $R_{TX}$, $R_{RX}$, $R_{Del}$ aus Tensorentfaltungen $H_{(1)}$, $H_{(2)}$, $H_{(3)}$ eines mehrdimensionalen Arrays oder Tensors, das/der einen Kanalimpuls oder einen Frequenzgang auf einer sendeseitigen, empfangsseitigen und über eine Verzögerungsaufspreizung eines drahtlosen Multiple-Input, Multiple-Output-Übertragungssystems (1, 20) beschreibt, das eine Vielzahl von $N_{TX}$ Sendeantennen und eine Vielzahl von $N_{RX}$ Empfangsantennen aufweist, für einen definierten Zeitraum, wobei die Kanalkorrelationsmatrizen eine sendeseitige Matrix, eine empfangsseitige Matrix und eine Verzögerungsaufspreizung-Matrix umfasst, wobei die Tensorentfaltungen das Kanalverhalten bei Single-Input Multiple-Output, Multiple-Input Single-Output bzw. Verzögerungsaufspreizung wie folgt beschreiben:

$$H_{(1)} = \begin{bmatrix} h_{111} & \cdots & h_{11D} & h_{121} & \cdots & h_{12D} & \cdots & h_{1N_{Tx}1} & \cdots & h_{1N_{Tx}D} \\ \vdots & \ddots & \vdots & \vdots & \ddots & \vdots & & \vdots & \ddots & \vdots \\ h_{N_{Rx}11} & \cdots & h_{N_{Rx}1D} & h_{N_{Rx}2D} & \cdots & h_{N_{Rx}2D} & \cdots & h_{N_{Rx}N_{Tx}1} & \cdots & h_{N_{Rx}N_{Tx}D} \end{bmatrix}$$

$$H_{(2)} = \begin{bmatrix} h_{111} & \cdots & h_{N_{Rx}11} & h_{112} & \cdots & h_{N_{Rx}12} & \cdots & h_{11D} & \cdots & h_{N_{Rx}1D} \\ \vdots & \ddots & \vdots & \vdots & \ddots & \vdots & & \vdots & \ddots & \vdots \\ h_{1N_{Tx}1} & \cdots & h_{N_{Rx}N_{Tx}1} & h_{1N_{Tx}2} & \cdots & h_{N_{Rx}N_{Tx}2} & \cdots & h_{1N_{Tx}D} & \cdots & h_{N_{Rx}N_{Tx}D} \end{bmatrix}$$

$$H_{(3)} = \begin{bmatrix} h_{111} & \cdots & h_{1N_{Tx}1} & h_{211} & \cdots & h_{2N_{Tx}1} & \cdots & h_{N_{Rx}11} & \cdots & h_{N_{Rx}N_{Tx}1} \\ \vdots & \ddots & \vdots & \vdots & \ddots & \vdots & & \vdots & \ddots & \vdots \\ h_{11D} & \cdots & h_{1N_{Tx}D} & h_{21D} & \cdots & h_{2N_{Tx}D} & \cdots & h_{N_{Rx}1D} & \cdots & h_{N_{Rx}N_{Tx}D} \end{bmatrix}$$

   Eigenzerlegen jeder der Korrelationsmatrizen, um eine eigenzerlegte sendeseitige Matrix, eine eigenzerlegte empfangsseitige Matrix und eine eigenzerlegte verzögerungsaufgespreizte Matrix zu generieren;
   Extrahieren von sendeseitigen, empfangsseitigen und verzögerungsaufgespreizten Eigenvektoren aus den eigenzerlegten sendeseitigen, empfangsseitigen und verzögerungsaufgespreizten Korrelationsmatrizen;
   Generieren (16) von differentiellen Unitaritätsmaßen unter Verwendung von sendeseitigen, empfangsseitigen und verzögerungsaufgespreizten Eigenvektoren, die zu unterschiedlichen Zeitpunkten extrahiert werden, wobei das Generieren von differentiellen Unitaritätsmaßen das Generieren einer Anzeige von Änderungen in der Unitarität der jeweiligen Eigenbasen, wie sie aus den generierten differentiellen Unitaritätsmaßen bestimmt werden, umfasst; und
   Generieren einer Ausgabe, die die Anwesenheit oder die Aktivität in einem definierten Raum anzeigt, basierend auf den differentiellen Unitaritätsmaßen.

2. Verfahren nach Anspruch 1, wobei das Generieren der Ausgabe, die die Anwesenheit oder die Aktivität anzeigt, das Generieren einer Anzeige einer Anzahl von Menschen innerhalb des definierten Raums (2) umfasst.

3. Verfahren nach Anspruch 1 oder Anspruch 2, wobei das Generieren der Ausgabe, die die Anwesenheit oder die Aktivität anzeigt, das Generieren einer Anzeige eines Aktivitätsniveaus von Menschen innerhalb des definierten Raums (2) umfasst.

4. Verfahren nach einem der vorstehenden Ansprüche, das ferner das Erzeugen von Kanalimpuls- oder Frequenzgangdaten aus Kanalzustandsinformationen für das drahtlose Übertragungssystem (1, 20) umfasst.

5. Verfahren nach Anspruch 4, das ferner das Sammeln der Kanalzustandsinformationen umfasst.

6. Verfahren nach Anspruch 4 oder Anspruch 5, das ferner das Zusammenstellen der Kanalzustandsinformationen in dem mehrdimensionalen Array oder Tensor umfasst.

7. Verfahren nach einem der vorstehenden Ansprüche, wobei die Ausgabe, die die Anwesenheit oder die Aktivität anzeigt, unter Verwendung von Tools des maschinellen Lernens generiert wird.

8. Verfahren nach einem der vorstehenden Ansprüche, das ferner das Definieren des definierten Zeitraums umfasst.

9. Verfahren nach einem der vorstehenden Ansprüche, das ferner das Generieren eines Modells für maschinelles Lernen umfasst.

10. Verfahren nach einem der vorstehenden Ansprüche, wobei das Extrahieren der empfangsseitigen, sendeseitigen und verzögerungsaufgespreizten Eigenvektoren das Auswählen von Eigenvektoren mit den stärksten Eigenwerten aus den eigenzerlegten Korrelationsmatrizen umfasst.

11. Verfahren nach einem der vorstehenden Ansprüche, wobei die statistischen Kanalkorrelationsmatrizen durch Tensorentfaltung generiert werden.

12. Verfahren nach einem der vorstehenden Ansprüche, wobei das Generieren von differentiellen Unitaritätsmaßen empfangsseitige, sendeseitige und verzögerungsaufgespreizte Eigenvektoren zu benachbarten Zeitpunkten verwendet.

13. Verfahren nach einem der Ansprüche 1 bis 11, wobei das Generieren von differentiellen Unitaritätsmaßen das Generieren von mindestens einigen differentiellen Unitaritätsmaßen unter Verwendung empfangsseitiger, sendeseitiger und verzögerungsaufgespreizter Eigenvektoren zu nicht benachbarten Zeitpunkten umfasst.

14. Computerprogramm mit Befehlen, die bei Ausführung durch einen Computer den Computer so steuern, dass er das Verfahren nach einem der vorstehenden Ansprüche ausführt.

15. Vorrichtung, die dafür ausgelegt ist, das Verfahren nach einem der Ansprüche 1 bis 13 durchzuführen.

**Revendications**

1. Procédé comprenant :

la génération de matrices de corrélation des canaux statistiques $R_{TX}$, $R_{RX}$, $R_{Del}$, à partir de développements de tenseurs $H_{(1)}$, $H_{(2)}$, $H_{(3)}$ d'un réseau ou tenseur multidimensionnel décrivant une réponse impulsionnelle ou en fréquence de canaux au niveau d'un côté émission, d'un côté réception et de part et d'autre d'un étalement de retard d'un système de transmission sans fil à entrées multiples et sorties multiples (1, 20) ayant une pluralité de $N_{TX}$ antennes d'émission et une pluralité de $N_{RX}$ antennes de réception pendant une période définie, dans lequel les matrices de corrélation des canaux comprend une matrice côté émission, une matrice côté réception et une matrice d'étalement de retard, dans lequel les développements de tenseurs décrivent un comportement de canaux en entrée unique et sorties multiples, en entrées multiples et sortie unique et en étalement de retard, respectivement, comme suit :

$$H_{(1)} = \begin{bmatrix} h_{111} & \cdots & h_{11D} & h_{121} & \cdots & h_{12D} & \cdots & h_{1N_{Tx}1} & \cdots & h_{1N_{Tx}D} \\ \vdots & \ddots & \vdots & \vdots & \ddots & \vdots & & \vdots & \ddots & \vdots \\ h_{N_{Rx}11} & \cdots & h_{N_{Rx}1D} & h_{N_{Rx}2D} & \cdots & h_{N_{Rx}2D} & \cdots & h_{N_{Rx}N_{Tx}1} & \cdots & h_{N_{Rx}N_{Tx}D} \end{bmatrix}$$

$$H_{(2)} = \begin{bmatrix} h_{111} & \cdots & h_{N_{Rx}11} & h_{112} & \cdots & h_{N_{Rx}12} & \cdots & h_{11D} & \cdots & h_{N_{Rx}1D} \\ \vdots & \ddots & \vdots & \vdots & \ddots & \vdots & & \vdots & \ddots & \vdots \\ h_{1N_{Tx}1} & \cdots & h_{N_{Rx}N_{Tx}1} & h_{1N_{Tx}2} & \cdots & h_{N_{Rx}N_{Tx}2} & \cdots & h_{1N_{Tx}D} & \cdots & h_{N_{Rx}N_{Tx}D} \end{bmatrix}$$

$$H_{(3)} = \begin{bmatrix} h_{111} & \cdots & h_{1N_{Tx}1} & h_{211} & \cdots & h_{2N_{Tx}1} & \cdots & h_{N_{Rx}11} & \cdots & h_{N_{Rx}N_{Tx}1} \\ \vdots & \ddots & \vdots & \vdots & \ddots & \vdots & & \vdots & \ddots & \vdots \\ h_{11D} & \cdots & h_{1N_{Tx}D} & h_{21D} & \cdots & h_{2N_{Tx}D} & \cdots & h_{N_{Rx}1D} & \cdots & h_{N_{Rx}N_{Tx}D} \end{bmatrix}$$

la décomposition en valeurs propres de chacune desdites matrices de corrélation pour générer une matrice côté émission décomposée en valeurs propres, une matrice côté réception décomposée en valeurs propres et une matrice d'étalement de retard décomposée en valeurs propres ;

l'extraction de vecteurs propres côté émission, côté réception et d'étalement de retard à partir des matrices de corrélation côté émission, côté réception et d'étalement de retard décomposées en valeurs propres ;

la génération (16) de mesures d'unitarité différentielle à l'aide des vecteurs propres côté émission, côté réception et d'étalement de retard extraits à différents instants, dans lequel la génération de mesures d'unitarité différentielle comprend la génération d'une indication de variations unitaires de bases propres respectives telles que déterminées à partir des mesures d'unitarité différentielle générées ; et

la génération d'une sortie indicative d'une présence ou d'une activité au sein d'un espace défini sur la base desdites mesures d'unitarité différentielle.

2. Procédé selon la revendication 1, dans lequel la génération de la sortie indicative d'une présence ou d'une activité inclut la génération d'une indication d'un nombre d'êtres humains au sein de l'espace défini (2).

3. Procédé selon la revendication 1 ou la revendication 2, dans lequel la génération de la sortie indicative d'une présence ou d'une activité inclut la génération d'une indication d'un niveau d'activité d'êtres humains au sein de l'espace défini (2).

4. Procédé selon l'une quelconque des revendications précédentes, comprenant en outre la génération de données de réponse impulsionnelle ou en fréquence de canaux à partir d'informations d'état de canal pour le système de transmission sans fil (1, 20).

5. Procédé selon la revendication 4, comprenant en outre la collecte desdites informations d'état de canal.

6. Procédé selon la revendication 4 ou la revendication 5, comprenant en outre le regroupement desdites informations d'état de canal en le réseau ou tenseur multidimensionnel.

7. Procédé selon l'une quelconque des revendications précédentes, dans lequel la sortie indicative d'une présence ou d'une activité est générée à l'aide d'outil d'apprentissage automatique.

8. Procédé selon l'une quelconque des revendications précédentes, comprenant en outre la définition de ladite période définie.

9. Procédé selon l'une quelconque des revendications précédentes, comprenant en outre la génération d'un modèle d'apprentissage automatique.

10. Procédé selon l'une quelconque des revendications précédentes, dans lequel l'extraction des vecteurs propres côté réception, côté émission et d'étalement de retard comprend la sélection de vecteurs propres ayant les plus fortes valeurs propres à partir des matrices de corrélation décomposées en valeurs propres.

11. Procédé selon l'une quelconque des revendications précédentes, dans lequel les matrices de corrélation de canaux statistiques sont générées par développement de tenseurs.

12. Procédé selon l'une quelconque des revendications précédentes, dans lequel la génération de mesures d'unitarité différentielle utilise les vecteurs propres côté réception, côté émission et d'étalement de retard à des instants adjacents.

13. Procédé selon l'une quelconque des revendications 1 à 11, dans lequel la génération de mesures d'unitarité différentielle comprend la génération d'au moins certaines mesures d'unitarité différentielle à l'aide des vecteurs propres côté réception, côté émission et d'étalement de retard à des instants non adjacents.

14. Programme d'ordinateur comprenant des instructions qui, lors de leur exécution par un ordinateur, le commandent pour réaliser le procédé selon l'une quelconque des revendications précédentes.

15. Appareil configuré pour réaliser le procédé selon l'une quelconque des revendications 1 à 13.

Fig. 1

Fig. 2

**Fig. 3**

20

Sideways placement 2

31

**Fig. 4**

Diagonal placement (optimum)

32

**Fig. 5**

Sideways placement 1

Fig. 6

41

Diagonal placement (optimum)

Fig. 7

42

Fig. 8

50

*Fig. 9*

*Fig. 10*

*Fig. 11*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WANG WEI.** Device-Free Human Activity Recognition Using Commercial WiFi Devices. *IEEE Journal on Selected Areas in Communications,* May 2017, vol. 35 (5), 1118-1131